(19) Europäisches Patentamt / European Patent Office / Office européen des brevets

(11) **EP 1 782 771 A1**

(12) **EUROPEAN PATENT APPLICATION**
published in accordance with Art. 158(3) EPC

(43) Date of publication:
**09.05.2007 Bulletin 2007/19**

(21) Application number: **05765764.5**

(22) Date of filing: **14.07.2005**

(51) Int Cl.:
*A61F 7/08* (2006.01)    *C09K 5/16* (2006.01)

(86) International application number:
**PCT/JP2005/013000**

(87) International publication number:
**WO 2006/006647 (19.01.2006 Gazette 2006/03)**

(84) Designated Contracting States:
**DE FR GB**

(30) Priority: **14.07.2004  JP 2004207828**

(71) Applicant: **Mycoal Products Corporation
Tochigi-chi, Tochigi 328-0067 (JP)**

(72) Inventor: **DODO, Toshihiro; c/o MYCOAL
PRODUCTS CORPORATION,
32 80067 (JP)**

(74) Representative: **Thun, Clemens
Mitscherlich & Partner
Sonnenstrasse 33
80331 München (DE)**

(54) **ACTIVE IRON POWDER AND EXOTHERMAL ARTICLE**

(57)    There is provided an active iron powder which is suitable as a raw material of a heat generating body, is excellent in rising properties of a heat generating composition and is excellent in economy.

The invention is concerned with an active iron powder to be contained in a heat generating composition capable of generating heat upon contact with air, **characterized in that** an amount of wustite to be contained in an iron powder is from 5.01 to 50 % by weight in terms of an X-ray peak intensity ratio to iron.

FIG. 3

EP 1 782 771 A1

**Description**

[Technical Field]

The present invention relates to an active iron powder which is used in a heat generating body and to a heat generating composition and a heat generating body.

[Background Art]

As products to be used by making air (oxygen) act on a mixture of an iron powder and a reaction aid, etc., in general, throwaway body warmers and so-called oxygen-free scavengers which are installed in a packaging body of various foods and efficiently absorb oxygen in the packaging body, thereby preserving the freshness of foods are well known.

As metal powders to be used in these products, an iron powder is the most general, and as the reaction aid, salt, water, and the like are used. It is also well known that as a water retaining agent for carrying such a substance thereon, active carbon, vermiculite, diatomaceous earth, a wood meal, a water absorptive polymer, and the like are mixed and used.

A role of the iron powder in a throwaway body warmer is to utilize reaction heat as generated due to oxidation, thereby achieving the purpose. Accordingly, the performance of such a product is influenced by characteristics of the iron powder. In other words, if an iron powder having high activity is used, favorable products are produced.

In a throwaway body warmer, since what the temperature rises immediately after breaking the seal enhances a product value, it is desired to supply an iron powder having excellent exothermic rising characteristics (see, for example, Patent Documents 1 and 2).

Usually, in commercially available iron powders, the degree of reduction is strong, and the purity of iron is high. When used for a heat generating body, exothermic rising properties are insufficient, and it is attempted to improve the exothermic rising properties chiefly by adjusting the amount of addition of a carbon component such as active carbon. However, satisfactory results have not been obtained yet in view of the performance.

Then, an active iron powder whose surface has been modified by forming a fixed amount of a thin film of a conductive carbonaceous substance locally on the surface of an iron powder such that an oxidation reaction is promoted is known as a raw material for throwaway body warmers or oxygen-free scavengers.

[Patent Document 1] JP-A-53-60885
[Patent Document 2] JP-A-57-10673

[Disclosure of the Invention]

[Problems that the Invention is to Solve]

However, according to the method for forming a fixed amount of a thin film of a conductive carbonaceous substance locally on the surface of iron, though the exothermic rising properties of a heat generating composition are improved, an effect thereof is insufficient, and a production step thereof becomes complicated. Thus, there was involved a problem in view of costs.

An object of the invention is to provide an active iron powder which is suitable as a raw material of a heat generating body, is excellent in rising properties of a heat generating composition and is excellent in economy.

[Means for Solving the Problems]

The present inventors made extensive and intensive investigations, examined a production process of an iron powder and examined exothermic rising properties of a heat generating composition by treating a reduced iron powder with an oxidizing gas and using various iron powders as prepared by changing their conditions and compounding, and the like. As a result, it has been noted that in an iron powder having favorable exothermic rising properties of a heat generating composition, a wustite phase (FeO) is present. Then, as a result of examining an existent ratio of wustite and iron in terms of an X-ray peak intensity ratio, it has been found that there is a relation in which when an intensity ratio of wustite drops, the exothermic rising properties become worse and that in a sample wherein wustite is present, the exothermic rising properties are favorable.

As set forth in claim 1, an active iron powder of the invention is an active iron powder to be contained in a heat generating composition capable of generating heat upon contact with air, which is characterized in that an amount of wustite to be contained in an iron powder is from 5.01 to 50 % by weight in terms of an X-ray peak intensity ratio to iron.

Also, an active iron powder as set forth in claim 2 is characterized in that in the active iron powder as set forth in claim 1, the active iron powder is at least one member selected from a reduced iron powder, an atomized iron powder, and an iron powder comprising particles, a surface of each of which is at least partially covered by a conductive carbonaceous substance.

As set forth in claim 3, a heat generating composition of the invention is the heat generating composition as set forth in claim 1 containing as essential components containing an iron active powder according to claim 1, a carbon component, a reaction accelerator and water, characterized in that the active iron powder accounts for from 30 to 100 % by weight of the iron powder in the heat generating composition.

As set forth in claim 4, a heat generating body of the invention is characterized by containing the heat generating composition as set forth in claim 3.

Also, the heat generating body as set forth in claim 5 is characterized in that in the heat generating body as set forth in claim 4, the heat generating body has fixing means in at least a part thereof.

Also, the heat generating body as set forth in claim 6 is characterized in that in the heat generating body as set forth in claim 5, the fixing means is an adhesive layer; and that the adhesive layer contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

[Advantages of the Invention]

As is clear from the foregoing description, by forming wustite at least on the surface of an iron powder as produced by reduction of iron oxide or at least on the surface of a commercially available iron powder or an iron powder as produced as an intermediate stage product of processing and production such that an amount of wustite is from 5.01 to 50 % by weight in terms of an X-ray peak intensity ratio to iron, an active iron powder for heat generating body which is not only suitable as a raw material of a heat generating body but also excellent in exothermic rising properties and excellent in economy is obtainable.

[Best Modes for Carrying Out the Invention]

The active iron powder of the invention is an active iron powder which is contained in a heat generating composition capable of generating heat upon contact with air, in which an amount of wustite to be contained in an iron powder is from 5.01 to 50 % by weight in terms of an X-ray peak intensity ratio to iron.

When the amount of wustite is large, a gas was generated so that there was encountered a problem such that an outer bag which is an air-impermeable accommodating bag is swollen during the storage. However, by using a small amount of a hydrogen gas formation inhibitor, this problem was solved.

Examples of the iron powder include a cast iron powder, an atomized iron powder, an electrolyzed iron powder, a reduced iron power, an iron powder whose surface is partially covered by a conductive carbonaceous substance, and iron alloys thereof.

An X-ray analysis of a wustite phase was evaluated by using an X-ray diffraction device, and the amount of wustite was evaluated as a ratio between an integrated intensity of peaks on a (110) plane of iron and an integrated intensity of peaks of a (220) plane of wustite.

In the invention, a wustite region is present at least one the surface of this iron powder, and an amount of wustite which is contained in the iron powder is from 5.01 to 50 % by weight, preferably from 5.01 to 30 % by weight, more preferably from 7 to 30 % by weight, and further preferably from 7 to 20 % by weight in terms of an X-ray peak intensity ratio to iron.

When the amount of wustite is less than 5.01 % by weight, it is impossible to contribute to the exothermic rising properties of a heat generating composition, whereas when it exceeds 50 % by weight, a special effect is not promoted so that an exothermic time of a heat generating composition or a heat generating body becomes short.

Furthermore, in the active iron powder of the invention, wustite and/or an iron oxide other than wustite may be present. Examples of the iron oxide include irons containing oxygen such as oxides, hydroxides and oxyhydroxides of iron.

Usually, in the reduced iron powder, the presence of wustite which is an iron oxide may possibly be formed due to air oxidation during the storage, etc. However, the wustite fraction in such a state does not contribute to the exothermic rising properties of a heat generating composition.

By using the active iron powder of the invention as an iron powder for heat generating body, not only exothermic characteristics, especially initial rising characteristics can be improved, but also the amounts of a combustion improver and active carbon in the active components can be reduced.

That is, the invention is to use an iron powder which is generally produced at present and modify its function into an active iron powder suitable as a raw material of a heat generating body.

The iron powder or active iron powder may contain a metal other than iron, a semiconductor, and an oxide thereof.

The "iron alloy powder" as referred to herein is an iron alloy powder containing 50 % or more of iron. The alloy component is not particularly limited so far as it is a metal component including semiconductors other than iron and the iron component functions as a component of the heat generating composition. Examples thereof include silicon, zinc, aluminum, magnesium, manganese, nickel, and copper.

The iron powder may be an iron powder which contains a carbon component and/or is partially covered by a carbon component.

In particular, an iron powder or an iron alloy powder wherein the iron component is an iron powder or an iron alloy powder, the surface of which is partially covered by from 0.3 to 3.0 % by weight of a conductive carbonaceous substance, is useful.

As the metal oxide other than iron oxide in the iron component which contains oxygen and/or is covered by oxygen, any substance may be employed so far as it does not hinder the oxidation of iron by an oxidizing gas. Examples thereof include manganese dioxide and cupric oxide.

The heat generating composition contains, as essential components, an iron powder, a carbon component, a reaction accelerator and water, and the active iron powder accounts for from 30 to 100 % by weight of the iron powder in the heat generating composition.

As the water, one from a proper source may be employed. Its purity and kind and the like are not particularly limited.

In the case of the heat generating composition, the content of water is preferably from 1 to 70 % by weight, more preferably from 1 to 60 % by weight, further preferably from 7 to 60 % by weight, still further preferably from 10 to 50 % by weight, and even further preferably from 20 to 30 % by weight of the heat generating composition.

Furthermore, in the case of the reaction mixture or heat generating mixture prior to the contact treatment with an oxidizing gas, the content of water is preferably from 0.5 to 20 % by weight, more preferably from 1 to 20 % by weight, further preferably from 3 to 20 % by weight, and still further preferably from 4 to 15 % by weight of the reaction mixture or heat generating mixture.

The carbon component is not particularly limited so far as it contains carbon as a component. Examples thereof include carbon black, graphite, active carbon, carbon nanotubes, carbon nanohorns, and flullerenes. Carbon which has become conductive by doping or the like is also employable. There are enumerated active carbons as prepared from coconut shell, wood, charcoal, coal, bone carbon, etc. and carbons as prepared from other raw materials such as animal products, natural gases, fats, oils, and resins. In particular, active carbons having an adsorption retaining ability are preferable.

Furthermore, it is not always required that the carbon component is present alone. In the case where an iron powder containing the carbon component and/or covered by the carbon component is used in the heat generating composition, it is to be noted that the heat generating composition contains the carbon component even though the carbon component is not present alone.

The reaction accelerator is not particularly limited so far as it is able to promote the reaction of the heat generating substance. Examples thereof include metal halides, nitrates, acetates, carbonates, and metal sulfates. Examples of metal halides include sodium chloride, potassium chloride, magnetic chloride, calcium chloride, ferrous chloride, ferric chloride, sodium bromide, potassium bromide, ferrous bromide, ferric bromide, sodium iodide, and potassium iodide. Examples of nitrates include sodium nitrate and potassium nitrate. Examples of acetates include sodium acetate. Examples of carbonates include ferrous carbonate. Examples of metal sulfates include potassium sulfate, sodium sulfate, and ferrous sulfate.

The production process of an active iron powder according to the invention is not limited so far as the amount of wustite to be contained in the iron powder can be controlled at from 5.01 to 50 % by weight in terms of an X-ray peak intensity ratio to iron. Examples thereof include a contact treatment with an oxidizing gas in which a reaction mixture having components of a heat generating composition mixed therein or a heat generating composition is brought into continuous or intermittent contact with an oxidizing gas (for example, oxygen and air) in an oxidizing gas atmosphere or by blowing an oxidizing gas, or the like, thereby partially oxidizing the iron component.

A method for determining a degree of oxidation is not limited. Examples thereof include a method of determining a degree of contact of the reaction mixture or heat generating composition with an oxidizing gas by a water mobility value of the reaction mixture or heat generating composition, a contact time with an oxidizing gas, an exothermic temperature rise rate at the time of contact, an exothermic temperature at the time of contact, a maximum exothermic temperature at the time of contact, a prescribed temperature as dropped after reaching a maximum exothermic temperature at the time of contact, or a combination thereof, thereby determining a degree of oxidation.

In addition, the following can be specifically enumerated.

1. A production process by reducing a mill scale or an ore to be used as a raw material of an iron powder at a temperature of not higher than about 1, 300 °C by using a reducing agent such as hydrogen, charcoal, and coke, coarsely pulverizing the reduced cake by a hammer mill, a jaw crusher, etc., and then finely pulverizing it by a novorotor, a pulverizer, or a vibration bowl.

2. A production process by reducing an iron powder containing an iron oxide, thereby producing a partially oxidized iron powder.

3. A production process of a heat generating mixture by subjecting a reaction mixture of an iron powder, a reaction accelerator and water to a self-exothermic reaction in an oxidizing gas atmosphere to partially oxidize the iron powder, thereby containing an iron having an iron oxide film on the surface thereof.

4. A production process of a heat generating mixture by subjecting a reaction mixture of an iron powder, a reaction accelerator, an acidic substance and water to a self-exothermic reaction in an oxidizing gas atmosphere.

5. A production process of a heat generating mixture by subjecting a reaction mixture of an iron powder, a reaction accelerator, a carbon component and water to a self-exothermic reaction in an oxidizing gas atmosphere.

6. A production process of a heat generating mixture by subjecting a reaction mixture of an iron powder, a reaction accelerator, an acidic substance, a carbon component and water to a self-exothermic reaction in an oxidizing gas atmosphere.

7. A production process of a heat generating mixture containing a partially oxidized iron powder by containing other component than the foregoing components in the reaction mixture or heat generating mixture as set forth above in any one of 1 to 6 and carrying out the process as set forth above in any one of 1 to 6.

8. A production process of a heat generating mixture by carrying out the process as set forth above in any one of 1 to 7 under circumstances warmed at at least 10 °C higher than the circumferential temperature.

9. A production process of a heat generating mixture by carrying out the process as set forth above in any one of 1 to 8 by blowing an oxidizing gas.

10. A production process of a heat generating mixture by carrying out the process as set forth above in 9 by blowing an oxidizing gas warmed at at least 10 °C higher than the circumferential temperature.

11. A production process of a heat generating composition by carrying out the contact treatment with an oxidizing gas in the process as set forth above in any of 1 to 10 until the temperature exceeds a maximum temperature which is a maximum point of a temperature rise due to the exothermic reaction.

12. A production process of a heat generating mixture by carrying out the contact treatment with an oxidizing gas in the process as set forth above in any of 1 to 11 until the temperature exceeds a maximum temperature due to the exothermic reaction and further drops by at least 10 to 20 °C from the maximum temperature.

13. A production process of a heat generating composition by carrying out the contact treatment with an oxidizing gas in the process as set forth above in any of 1 to 11 until the temperature exceeds a maximum temperature which is a maximum point of a temperature rise due to the exothermic reaction and after intercepting the oxidizing gas, holding it until at least the temperature of the reaction mixture drops by at least 10 to 20 °C from the maximum temperature.

14. A production process of a heat generating mixture by treating the reaction mixture or heat generating mixture as set forth above in any one of 1 to 7 under oxidizing gas circumstances by regulating a temperature rise at 1 °C or higher.

In addition, a heat generating mixture may be formed by adding other components to the heat generating mixture and further carrying out the treatment with an oxidizing gas.

Incidentally, the circumstances of the reaction mixture at the time of the contact treatment with an oxidizing gas are not limited so far as under circumstances of 0 °C or higher, the reaction mixture is brought into contact with an oxidizing gas and a temperature rise of the reaction mixture is regulated at 1 °C or higher within 10 minutes. Examples thereof include a state that it is present in a vessel and a state that the reaction mixture is present in an air-permeable sheet-like material such as non-woven fabrics.

Furthermore, the contact treatment with an oxidizing gas may be carried out with stirring or without stirring and additonally under fluidization or non-fluidization and may be carried out in a batchwise system or continuous system.

Here, with respect to the state of the reaction mixture at the time of the contact treatment with an oxidizing gas, so far as the amount of wustite which is contained in the partially oxidized iron powder is from 5.01 to 50 % by weight in terms of an X-ray peak intensity ratio to iron, any of a standing state, a transfer state and a fluidizing state by stirring, etc. may be employed and properly selected. Furthermore, mixing of the respective components of the reaction mixture, heat generating mixture or heat generating composition and mixing at the time of adjusting the water content may be achieved in an oxidizing gas atmosphere or by blowing an oxidizing gas.

The heat generating composition is not limited so far as it is a heat generating composition comprising an iron powder containing from 30 to 100 % of an active iron powder and other essential components or a heat generating composition further containing components other than the essential components.

Examples of the production process of the heat generating composition include:

1) A production process by containing the active iron powder as produced by the process as set forth above in any one of 1 to 14 and the essential components other than the iron powder;

2) A production process by mixing the active iron powder as produced by the process as set forth above in any one of 1 to 14 and the essential components other than the iron powder;

3) A production process by mixing the active iron powder as produced by the process as set forth above in any one of 1 to 14 and an iron powder and further adding and mixing the essential components other than the iron powder; and

4) A process for producing a heat generating composition by adjusting the water content of the composition as produced in 1) to 3) and mixing.

Incidentally, a production process of a heat generating composition by contact treating a reaction mixture prepared by adding and mixing the essential components and other components with an oxidizing gas and then adjusting the water content is especially preferable.

By using the active iron powder of the invention as an iron powder, exothermic rising characteristics of the heat generating composition are improved so that it is possible to reduce the amount of a carbon component such as active carbon in the heat generating composition by 10 to 20 % or more. By reducing the amount of addition of the carbon component, it is possible to reduce costs.

Although a mechanism in which when the active iron powder of the invention is used as an iron powder for heat generating body, the exothermic rising properties of the heat generating composition are improved has not be elucidated in detail, it is assumed that because of a catalytic effect of wustite against oxidation of iron and contact between an oxidizing gas and the components to cause oxidation of the components, in particular, oxidation of an iron powder, not only an iron oxide film, i.e., an oxygen-containing film, is formed on the surface of the iron powder, but also the oxidized iron component is also adhered on the surface of active carbon, whereby hydrophilicity is imparted or improved on the both to cause coupling or structurization among the components through the mediation of water. Furthermore, the case where magnetite ($Fe_3O_4$) is present in the iron oxide film is preferable because it is excellent in conductivity. The case where hematite ($Fe_2O_3$) is present in the iron oxide film is also preferable because it becomes porous.

It is preferable that the heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

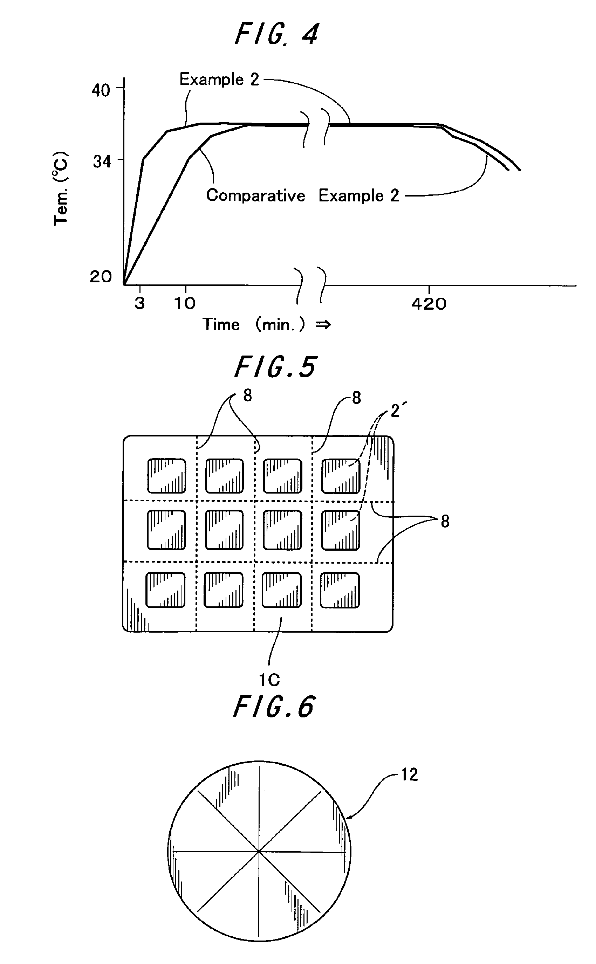

The "water mobility value" as referred to herein is a value showing an amount of surplus water which can transfer to the outside of the heat generating composition in water present in the heat generating composition. This water mobility value will be described below with reference to Figs. 6 to 10.

As shown in Fig. 6, a filter paper 12 of No. 2 (second class of JIS P3801) in which eight lines are drawn radiating from the central point with an interval of 45° is placed on a stainless steel plate 16 as shown in Figs. 7 and 8; a template 13 having a size of 150 mm in length x 100 mm in width and having a hollow cylindrical hole 14 having a size of 20 mm in inner diameter x 8 mm in height is placed in the center of the filter paper 12; a sample 15 is placed in the vicinity of the hollow cylindrical hole 14; and a stuffer plate 9 is moved on and along the template 13 and inserted into the hollow cylindrical hole 14 while stuffing the sample 15, thereby leveling the sample (force-in die molding).

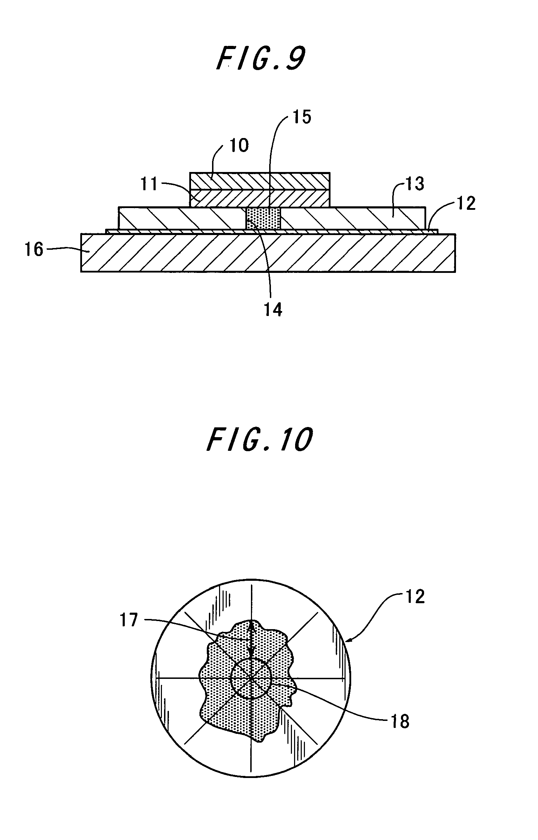

Next, as shown in Fig. 9, a non-water absorptive 70 $\mu$m-thick polyethylene film 11 is placed so as to cover the hole 14, and a flat plate 16 made of stainless steel having a size of 5 mm in thickness x 150 mm in length x 150 mm in width is further placed thereon and held for 5 minutes such that an exothermic reaction is not caused.

Thereafter, a shown in Fig. 10, the filter paper 12 is taken out, and an oozed-out locus of the water or aqueous solution is read as a distance 17 (unit: mm) from a periphery 18 as an edge of the hollow cylindrical hole to an oozed-out tip along the radiating lines. Similarly, a distance 17 from each of the lines is read, and eight values in total are obtained. Each of the eight values (a, b, c, d, e, f, g and h) which are read out is defined as a measured water content value. An arithmetic average value of the eight measured water content values is defined as a water content value (mm) of the sample.

Furthermore, the water content for the purpose of measuring a real water content value is defined as a compounded water content of the heat generating composition corresponding to the weight of the heat generating composition having a size of 20 mm in inner diameter x 8 mm in height or the like, similar measurement is conducted only with water corresponding to that water content, and a value as calculated in the same manner is defined as a real water content value (mm). A value obtained by dividing the water content value by the real water content value and then multiplying with 100 is a water mobility value.

That is, the water mobility value is represented by the following expression.

$$\text{(Water mobility value)} = \{[\text{Water content value (mm)}]/$$

$$[(\text{Real water content value (mm))}] \times 100$$

With respect to the same sample, five points are measured, and the five water mobility values are averaged, thereby defining an average value thereof as a water mobility value of the sample.

In the invention, the water mobility value (0 to 100) is preferably from 0.01 to 20, more preferably from 0.01 to 18, further preferably from 0.01 to 15, still further preferably from 0.01 to 13, even further preferably from 1 to 13, and even still further preferably from 3 to 13.

A heat generating composition having a water mobility value of less than 0.01 is insufficient in moldability. A heat generating composition having a water mobility value of from 0.01 to 50 has moldability and therefore, is a moldable heat generating composition. When the water mobility value exceeds 20, it is necessary that a part of water of the heat generating composition is removed by water absorption, dehydration, etc. That is, unless a part of water in the heat generating composition molded body is removed by water absorption, dehydration, etc. using a water absorptive packaging material, etc., a practical useful exothermic reaction is not caused. Incidentally, in the case where a water absorptive polymer having a low water absorption speed is used and although a high water mobility value is exhibited at the time of molding, after elapsing a certain period of time, a part of surplus water is taken in the water absorptive polymer, whereby the heat generating composition becomes in an exothermic state with a water mobility value of from 0.01 to 20, even a heat generating composition having a high water mobility value is dealt as a heat generating composition in which surplus water does not function as a barrier layer. In a heat generating composition having a water mobility value exceeding 50, surplus water is too much, the heat generating composition becomes in a slurry state and loses moldability, and the surplus water functions as a barrier layer. Thus, even upon contact with air as it is, an exothermic reaction is not caused.

Furthermore, the "water mobility value" as referred to herein is a value obtained by digitizing surplus water which is the water content capable of being easily and freely oozed out the system in water which is contained in the heat generating composition or mixture or the like. In a mixture in which some components of the heat generating composition or mixture or the like are mixed, the amount of the surplus water is variously changed depending the amount of a component having a water retaining ability such as a water retaining agent, a carbon component and a water absorptive polymer and wettability of each component, and therefore, it is every difficult to predict the water mobility value from the amount of addition of water. Accordingly, since the amount of surplus water of the heat generating composition or mixture of the like is determined from the water mobility value, by determining the amount of addition of water and the amount of other components, a heat generating composition or mixture or the like having a substantially fixed amount of surplus water is obtained with good reproducibility. That is, by previously examining the water mobility value and a composition ratio of a heat generating composition or mixture or the like, a heat generating composition or mixture or the like as compounded along that composition ratio has a water mobility value falling within a fixed range, namely, an amount of surplus water falling within a fixed range. Thus, it is possible to easily produce a variety of heat generating compositions such as a powdered heat generating composition which causes heat generation upon contact with air but does not have moldability, a heat generating composition which causes heat generation upon contact with air and has moldability, and a heat generating composition which, after discharging out a fixed amount of surplus water from the system by water absorption, etc., causes heat generation upon contact with air and has moldability. Accordingly, if the water mobility value is known, it is possible to note what state does the subject heat generating composition or mixture or the like take.

If the water mobility value is employed, it is possible to embody a desired state with good reproducibility by a simple measurement. Thus, it becomes possible to determine a component ratio of the heat generating composition on the basis of the water mobility value obtained by the measurement and the component ratio, thereby simply achieving actual production of a heat generating composition.

As a use example of the water mobility value, water (or a reaction accelerator aqueous solution) is added to and mixed with a mixture of specified amounts of heat generating composition components exclusive of water (or a reaction accelerator aqueous solution), thereby producing plural heat generating compositions having a different water content. Next, a water mobility value of each of the heat generating compositions is measured, thereby determining a relationship between the amount of addition of water (or a reaction accelerator aqueous solution) and a water mobility value.

A heat generating composition which has moldability and causes heat generation upon contact with air has a water mobility value of from 0.01 to 20. By determining a compounding ratio of the respective components therefrom to prepare a mixture in this compound ratio, a moldable heat generating composition in which water does not function as a barrier layer and which has moldability causes heat generation upon contact with air can be produced with good reproducibility. In this way, since surplus water is used as a connecting substance and a flocculant aid or a dry binding material is not used, reaction efficiency of the iron powder does not drop. Thus, an exothermic performance can be obtained in a small amount as compared with the case of using a flocculant aid or a dry binding material.

Incidentally, in the invention, what water does not function as a barrier layer and causes an exothermic reaction upon contact with air means that water in a heat generating composition does not function as a barrier layer which is an air intercepting layer and immediately after the production of a heat generating composition, comes into contact with air, thereby immediately causing an exothermic reaction.

By using a moldable heat generating composition containing this surplus water as a connecting substance, it becomes possible to produce, for example, a super thin and super flexible heat generating body having plural sectional exothermic parts of a heat generating composition molded body on a substantially planar substrate in a maximum width of preferably from 1 to 50 mm, and more preferably from 1 to 20 mm, or in a maximum diameter of preferably from 1 to 50 mm, and more preferably from 1 to 20 mm (in the case where two or more axes are present as in an ellipse, the major axis is dealt as a length, while the minor axis is dealt as a width).

The "surplus water" as referred to herein means water or an aqueous solution portion which is present excessively in the heat generating composition and easily transfers to the outside of the heat generating composition. The surplus water is defined as a water mobility value which is a value of water or a value of an aqueous solution portion sucked out from the heat generating composition, etc. by a filter paper. When the heat generating composition has an appropriate amount of surplus water, it is assumed that the surplus water causes hydration against hydrophilic groups in the components of the heat generating composition due to a bipolar mutual action or hydrogen bond, etc. and that it is present even in the surroundings of hydrophobic groups while having high structural properties.

This is connecting water as a connecting substance in some meaning. Besides, there is water in a state called as free water which can freely move. When the surplus water increases, the structure is softened, and the free water is found.

The "moldability" as referred to in the invention exhibits that a molded body of the heat generating composition having a cavity or concave die shape is formed by force-through molding using a trimming die having a cavity or cast molding using a concave die, whereby after molding including mold release, the molding shape of the heat generating composition molded body is held.

When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, the sealing can be achieved without causing cutting in seal. The presence of the spots causes insufficient sealing.

1) Measurement device:

   With respect to the measurement device, a stainless steel-made molding die (a plate having a size of 2 mm in thickness x 200 mm in length x 200 mm in width and having a cavity as treated by R5 in four corners of 60 mm in length x 40 mm in width in a central part thereof) and a fixable leveling plate are disposed above a travelable endless belt, and magnets (two magnets having a size of 12.5 mm in thickness x 24 mm in length x 24 mm in width are disposed in parallel) are disposed under the endless belt.
   The magnets should cover a region of the leveling plate and the vicinity thereof and a region larger than a region covered by a cut side (40 mm) vertical to the advancing direction of the cavity of the molding die.

2) Measurement method:

   With respect to the measurement method, a stainless steel plate having a size of 1 mm in thickness x 200 mm in length x 200 mm in width is placed on the endless belt of the measurement device, a polyethylene film having a size of 70 μm in thickness x 200 mm in length x 200 mm in width is placed thereon, and a stainless steel-made molding die is further placed thereon.
   Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1. 8 m/min. After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 $\mu$m is not present and the number of collapsed pieces of the heat generating composition molded body having a maximum length of from 300 to 800 $\mu$m is not more than 5, it is to be noted that the heat generating composition has moldability.

The moldability is an essential property for a heat generating composition to be used in the molding system. If the heat generating composition does not have moldability, it is impossible to produce a heat generating body by the molding system.

The "adjustment of the water content" as referred to herein means that after contact treating the heat generating mixture with an oxidizing gas, water or an aqueous solution of a reaction accelerator is added. Although the amount of addition of water or an aqueous solution of a reaction accelerator is not limited, examples thereof include the addition of a weight corresponding to a reduced weight by the contact treatment and the addition of a weight such that a desired water mobility value is obtained.

Whether or nor the adjustment of the water content is introduced may be properly determined depending upon the utility.

A method for measuring a temperature rise of the heat generating composition is as follows.

1) A heat generating composition is allowed to stand in a state that it is sealed in an air-impermeable outer bag for one hour under a condition that the circumferential temperature is 20 ± 1 °C.

2) A magnet is provided in the vicinity of a central part of the back side of a polyvinyl chloride-made supporting plate (3 mm in thickness x 600 mm in length x 600 mm in width) of a footed supporting table so as to cover a cavity shape of a molding die.

3) A temperature sensor is placed on the central part of the supporting plate.

4) A polyethylene film (25 $\mu$m in thickness x 250 mm in length x 200 mm in width) as provided with an adhesive layer having a thickness of about 80 $\mu$m is stuck onto the supporting plate via a sticky layer such that the center of the polyethylene film is positioned at the sensor.

5) The heat generating composition is taken out from the outer bag.

6) A template (250 mm in length x 200 mm in width) having a cavity (80 mm in length x 50 mm in width x 3 mm in height) is placed above the central part of the polyethylene film; a sample is placed in the vicinity of the cavity; a force-in die plate is moved along the template; the sample is charged into the cavity while stuffing; and the sample is leveled while stuffing along the template plane (force-in die molding), thereby filling the sample in the die. Next, the magnet beneath the supporting plate is removed, and the temperature measurement is started.

With respect to the measurement of the exothermic temperature, the temperature is measured for 10 minutes at a measurement timing of 2 seconds using a data collector, and exothermic rising properties are judged in terms of the temperature after elapsing 3 minutes.

The heat generation test of the heat generating body follows the JIS temperature characteristic test.

The "contact treatment with an oxidizing gas" as referred to herein is a method in which a mixture or heat generating composition having components of the heat generating composition mixed therein is brought into continuous or intermittent contact with an oxidizing gas (for example, oxygen and air) in an oxidizing gas atmosphere or by blowing an oxidizing gas or other means, thereby partially oxidizing the iron component. A method for determining a degree of oxidation is not limited. Examples thereof include a method in which a degree of contact of the mixture or heat generating composition with an oxidizing gas is determined by the water mobility value of the mixture or heat generating composition, the contact time with the oxidizing gas, the exothermic temperature rise rate at the time of contact, the exothermic temperature at the time of contact, the maximum exothermic temperature at the time of contact, a prescribed temperature as dropped after reaching the maximum exothermic temperature at the time of contact, or a combination thereof, thereby determining a degree of oxidation.

For examples, the following methods are preferable.

(1) A heat generating composition having a water mobility value of not more than 20 (for example, less than 0.01 or from 0.01 to 20) is exposed to air while fluidizing by stirring or the like to cause self heat generation, intercepted from air for a desired period of time until the temperature exceeds a maximum exothermic temperature and then returned to room temperature, thereby forming a heat generating composition. In particular, a contact treatment with an oxidizing gas by exposing a heat generating mixture or heat generating composition having a water mobility value of less than 0.01 to air while stirring, thereby causing self heat generation is preferable.

(2) A heat generating composition having a water mobility value exceeding 20 is brought into contact with air and intercepted from air for a desired period of time, thereby forming a heat generating composition.

(3) Water or a reaction accelerator aqueous solution is added to the heat generating composition as obtained in either one of (1) or (2), and the water content of the mixture is adjusted, followed by mixing to form a heat generating composition having a desired water mobility value. The weight of the water or reaction accelerator aqueous solution to be added for the purpose of adjusting the water content is not limited. Examples thereof include a weight as reduced against the weight of the mixture or heat generating composition prior to exposing to air, namely prior to causing self heat generation, or a weight corresponding to the weight exceeding it. If desired, the temperature state of the mixture and the heat generating composition may be controlled prior to the contact treatment and/or at the time of contact treatment by warming the mixture, warming the heat generating composition and warming a reaction vessel, heat insulation, cooling, or a combination thereof. In this way, a heat generating composition having remarkably excellent exothermic rising properties can be obtained.

As the "oxidizing gas" as referred to herein, any substance may be employed so far as it is gaseous and oxidizing. Examples thereof include an oxygen gas, air, and a mixed gas of an inert gas (for example, a nitrogen gas, an argon gas, and a helium gas) and oxygen. As the mixed gas, it is preferable that it contains 10 % or more of an oxygen gas. Of these, air is especially preferable.

So far as the atmosphere of the contact treatment region does not become deficient in oxygen and an oxidation reaction of the iron component is caused, a temperature of the oxidizing gas, a temperature of the contact treatment and a time of the contact treatment are not limited and may be properly determined depending upon the desire. The temperature of the oxidizing gas is preferably from 0 to 200 °C, more preferably from 10 to 150 °C, and further preferably from 20 to 100 °C; and the treatment time is preferably from one second to 10 minutes, more preferably from 5 seconds to 7 minutes, and further preferably from 15 seconds to 5 minutes. In the step, it is preferable that the reaction time is short.

The amount of the oxidizing gas to be used may be adjusted depending upon the kind of the oxidizing gas, the kind and particle size of the iron powder, the water content, the treatment temperature, the treatment method, and the like. In the case of using air, the amount of air is preferably from 1 to 1,000 liters/min per 200 g of the iron powder under one atmosphere at 100 °C. In the case of other oxidizing gas, the amount of the oxidizing gas may be reduced into the concentration of oxygen on the basis of the case of air.

If desired, an acidic substance or a peroxide may be added at the time of the contact treatment with an oxidizing gas. Examples of the peroxide include hydrogen peroxide and ozone. In the case of carrying out the treatment with an oxidizing gas in an open system, the treatment may be carried out in a lid-free vessel or in a manner such that an oxidizing gas such as air comes into a vessel through an air-permeable sheet-like material such as non-woven fabrics.

The "heat generating mixture" as referred to herein is a material obtained by subjecting a reaction mixture containing, as essential components, an iron powder, a carbon component, a reaction accelerator and water and having a water content of from 1 to 20 % by weight and a water mobility value of less than 0.01 to a contact treatment with an oxidizing gas under fluidization, thereby regulating a temperature rise at 1 °C or higher within 10 minutes. So far as some change is caused in the reaction mixture by the contact treatment with an oxidizing gas, the iron powder is not always required to be oxidized. However, it is preferable that the iron powder is oxidized. In that case, it is preferable that the iron powder becomes an active iron powder.

The "amount of wustite" as referred to herein is an amount expressed by % according to the following expression from an integrated intensity of peaks of a (110) plane of iron ($\alpha$Fe) and an integrated intensity of peaks of a (220) plane of FeO (wustite) by using an X-ray diffraction device.

$$\text{[Amount of wustite (\%)]} = 100 \times \text{KFeO}/(\text{K}\alpha\text{Fe})$$

KFeO: Integrated intensity of peaks of a (220) plane of FeO (wustite)
K$\alpha$Fe: Integrated intensity of peaks of a (110) plane of iron ($\alpha$Fe)

The "active iron powder" as referred to herein is an iron powder having a content of wustite of from 5.01 to 50 % by weight. Furthermore, in the case where the active iron powder is prepared by using a mixture containing at least any one of an iron powder and other essential components (for example, a carbon component, a reaction accelerator, and water) and in the case where the amount of the active iron powder of the iron powder in the heat generating composition of the heat generating body is determined, the iron powder is separated from the mixture or the heat generating composition of the heat generating body after the preparation by using a magnet, etc. and used as a sample; the amount of wustite is determined by using an X-ray diffraction device; and if the amount of wustite falls within the range of from 2 to 50 % by weight, the resulting iron powder is defined to be an active iron powder.

The "heat generating mixture" as referred to herein is a material obtained by subjecting a reaction mixture containing, as essential components, an iron powder, a carbon component, a reaction accelerator and water and having a water content of from 1 to 30 % by weight and a water mobility value of less than 0.01 to a contact treatment with an oxidizing gas under fluidization and holding a temperature of the reaction mixture after the contact at 40 °C or higher for 2 seconds or more. So far as some change in characteristics is caused in the reaction mixture by the contact treatment with an oxidizing gas, the iron powder is not always required to be oxidized. However, it is preferable that the iron powder is oxidized. In that case, it is preferable that the iron powder becomes an active iron powder.

Furthermore, in the case where a heat generating body using a heat generating composition in which the active iron powder of the invention is used is required to be stored over a long period of time, by adding a hydrogen formation inhibitor to the heat generating composition, it is possible to store the heat generating body without causing swelling over a long period of time. After the storage over a long period of time, a heat generating body having excellent initial rising characteristics can be used.

The heat generating body of the invention is a heat generating body capable of generating heat upon contact with oxygen in air, and a heat generating body is formed by accommodating the foregoing heat generating composition containing an active iron powder in an air-permeable accommodating bag. In addition, the heat generating body may have plural exothermic parts by heat sealing among heat generating compositions.
Furthermore, the heat generating body may be sealed in an air-impermeable accommodating bag for the purpose of storage or transportation.

Although the production process of the heat generating body is not limited, the following production processes are enumerated.

1) Filling system:

The filling system is a method for coupling an end of a substrate or a partition part by an adhesive, sewing processing, or an appropriate system such a heat seal system to form a bag, filling a heat generating composition in the bag, and then bonding the end of the bag. As a process for producing a compartmentalized heat generating body by the filling system, there is enumerated a continuous formation method in which by using a longitudinal substrate and a rotary heat compressioning unit capable of heat sealing a desired partition part and the periphery of the substrate, the periphery of the longitudinal substrate and a necessary part of the partitioned as disposed opposite to each other via the heat compression unit are heated sealed, and at the same time, an air-permeable heat generating body is fed into a compartment formed of space between the substrates and subjected to a seal treatment, and the formation of a next compartment is started while bonding an end of the body warmer by this seal treatment.

2) Pocket system:

As disclosed in JP-T-11-508786, the pocket system is a process for producing a heat generating body in which a pocket is prepared in advance on a substrate by thermal molding, mechanical embossing, vacuum embossing, or other tolerable means, a heat generating composition and its compressed body, etc. are filled in the pocket, the pocket is covered by another substrate, and the surroundings of the two substrates are coupled.

3) Molding system:

The molding system is a process for producing a heat generating body in which a moldable heat generating composition is molded into a desired shape by a force-through molding method using a trimming die or a cast molding method using a casting mold, the molded body is laminated on a substantially planar substrate not having an accommodating pocket, etc., and another substrate is covered thereon, followed by sealing.
The "force-through molding method" as referred to herein means a continuous formation method in which by using a molding machine for laminating a heat generating composition molded body having a trimming die shape on a longitudinal substrate by using a trimming die and a rotary seal unit capable of covering the laminate by a longitudinal covering material and sealing (by heat seal, compression seal, or heat compression seal) a desired sectioned part and the surroundings of the substrate and the covering material, the surroundings of the heat generating composition molded body and a necessary part of the sectioned part are heat sealed via the seal unit and subjected to a seal treatment.

Furthermore, the "cast molding method" as referred to herein means a molding method for laminating a heat generating composition molded body on a longitudinal substance by filling in a casting mold having a concave and transferring it into a substrate.

In the continuous case, there is enumerated a continuous formation method in which by using a molding machine for laminating a heat generating molding molded body on a longitudinal substrate by filling in a concave and transferring into a substrate by a drum-type body of rotation and a rotary seal unit capable of covering the laminate by a longitudinal covering material and sealing (by heat seal, compression seal, or heat compression seal) a desired sectioned part and the surroundings of the substrate and the covering material, the surroundings of the heat generating composition molded body and a necessary part of the sectioned part are heat sealed via the seal unit and subjected to a seal treatment.

Furthermore, in producing a heat generating body using the heat generating composition of the invention according to the foregoing methods or other methods, a magnet may be used. By using a magnet, it becomes possible to easily achieve accommodation of the heat generating composition in a bag or a mold and separation of the molded body from the mold, thereby making it easier to mold a heat generating composition molded body or produce a heat generating body.

The fixing means is not limited so far as it has capability for fixing a thermal packaging body for joint surroundings or a material having an exothermic part to a prescribed part.

As the fixing means, an adhesive layer, a hook and eye, a hook and button, a hook and loop fastener such as Velcro, a magnet, a band, a string, and combination thereof can be arbitrarily used.

Incidentally, in the case of a band, fixing means for adjustment may be further constructed by a combination of a hook and loop fastener and an adhesive layer.

Here, the "hook and loop fastener" as referred to herein has a fastening function by a combination of a loop as a female fastener with a male fastener capable of fastening the female fastener thereto, which is known as trade names such as Magic Tape (a registered trademark), Magic Fastener (a registered trademark), Velcro Fastener, and Hook and Loop Tape. Examples of the material having a loop function include non-woven fabrics and woven fabrics of napped or hole-containing yarns. Such a material having a loop function (female fastener function) may be covered on the surface of a paddling forming the band, or the band may be constructed of such a material itself. Although the hook member which is the male fastener member is not particularly limited, examples thereof include hook members formed of a polyolefin based resin (for example, polyethylene and polypropylene), a polyamide, a polyester, etc. Although the shape of the hook is not particularly limited, a hook having a cross-sectional shape such as an I type, an inverted L type, an inverted J type, and a so-called mushroom type is preferable because it is easily hooked by the loop and does not give an extreme stimulus to the skin. Incidentally, the hook may be adhered to the entire area of a fastening tape, and only the hook may be used as a fastening tape while omitting a tape substrate.

The adhesive layer may contain at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

The adhesive of the invention is classified into a non-hydrophilic adhesive, a mixed adhesive, and a hydrophilic adhesive (for example, a gel).

The adhesive constituting the adhesive layer is not limited so far as it has an adhesive strength necessary for adhering to the skin or clothes. Adhesives of every form such as a solvent based adhesive, an aqueous adhesive, an emulsion type adhesive, a hot melt type adhesive, a reactive adhesive, a pressure-sensitive adhesive, a non-hydrophilic adhesive, and a hydrophilic adhesive are employable.

The adhesive layer includes one layer of a non-hydrophilic adhesive constituted of the non-hydrophilic adhesive and non-hydrophilic adhesive layers constituted of the non-hydrophilic adhesive.

It is to be noted that a material whose water absorption properties are improving by containing a water absorptive polymer or a water retaining agent in the non-hydrophilic adhesive layer is dealt as the non-hydrophilic adhesive layer.

A hot melt based adhesive may be provided between the hydrophilic adhesive layer and a substrate or a covering material.

Furthermore, in the case where the hydrophilic adhesive is provided in a thermal packaging body for joint surroundings, there is no limitation. After seal treating a thermal packaging body for joint surroundings, a hydrophilic adhesive layer may be provided in the thermal packaging body for joint surroundings.

Furthermore, the adhesive layer may or may not have air permeability and may be properly selected depending upon the utility. With respect to the air permeability, the adhesive layer may be air-permeable as a whole. Examples thereof include an adhesive layer having air permeability as a whole of a region in which an adhesive is partially present and a portion where no adhesive is present is partially present.

In laminating an adhesive on an air-permeable substrate and/or a covering material in a stratiform state as it is, examples of a method for keeping its air permeability include a method in which an adhesive layer is partially laminated by printing or transferring an adhesive, thereby forming a non-laminated part as an air-permeable part; a method in which an adhesive is transferred in one direction while drawing a circle in a filament-like form or properly moved in the two-dimensional directions by transferring in a zigzag manner, whereby a space of the filament-like adhesive keeps air permeability or moisture permeability or the adhesive is foamed; and a method for forming a layer by a melt blow system.

Examples of the adhesive which constitutes the non-hydrophilic adhesive layer include acrylic adhesives, polyvinyl acetate based adhesives (for example, vinyl acetate resin based emulsions and ethylene-vinyl acetate resin based holt melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, butyl rubber based adhesives, silicone rubber based adhesives, styrene based adhesives (for example, styrene based hot melt adhesives), rubber based adhesives, and silicone based adhesives. Of these, rubber based adhesives, acrylic adhesives, and adhesives containing a hot melt based polymer substance for the reasons that they are high in the adhesive strength, are cheap, are good in long-term stability, and are small in reduction of the adhesive strength even by providing heat.

In addition to the base polymer, if desired, the adhesive may be compounded with other components such as tackifiers (for example, petroleum resins represented by rosins, chroman-indene resins, hydrogenated petroleum resins, maleic anhydride-modified rosins, rosin derivatives, and C-5 based petroleum resins), phenol based tackifiers (especially, tackifiers having an aniline point of not higher than 50 °C; for example, terpene phenol based resins, rosin phenol based resins, and alkylphenol based resins), softeners (for example, coconut oil, castor oil, olive oil, camellia oil, and liquid paraffin), softeners, anti-aging agents, fillers, aggregates, adhesion adjusting agents, adhesion modifiers, coloring agents, anti-foaming agents, thickeners, and modifiers, thereby improving performance such as an improvement in adhesion to nylon-made clothes and mixed yarn clothes.

Examples of the hot melt based adhesive include known hot melt based adhesives imparted with adhesion. Specific examples thereof include styrene based adhesives made of, as a base polymer, an A-B-A type block copolymer (for example, SIS, SBS, SEBS, and SIPS), vinyl chloride based adhesives made of, as a base polymer, a vinyl chloride resin, polyester based adhesives made of, as a base polymer, a polyester, polyamide based adhesives made of, as a base polymer, a polyamide, acrylic adhesives made of, as a base polymer, an acrylic resin, polyolefin based adhesives made of, as a base polymer, a polyolefin (for example, polyethylene, super low density polyethylene, polypropylene, ethylene-$\alpha$-olefin copolymers, and ethylene-vinyl acetate copolymers), 1,2-polybutadiene based adhesives made of, as a base polymer, 1, 2-polybutadiene, and polyurethane based adhesives made of, as a base polymer, polyurethane; adhesives made of a modified body of the foregoing adhesive whose adhesion is improved or whose stability is changed; and mixtures of two or more kinds of these adhesives. Adhesive layers constituted of a foamed adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an $\alpha$-olefin. Also, polymers resulting from adding a diene (for example, butadiene and isoprene) as other monomer thereto may be employed.

The $\alpha$-olefin is not limited so far as it is a monomer having a double bond in the terminal thereof. Examples thereof include propylene, butene, heptane, hexene, and octene.

The "aromatic hot melt based adhesive" as referred to herein is a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound A such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS), and mixtures thereof.

As a countermeasure for preventing a lowering of adhesive strength caused due to an increase of water of the non-hydrophilic adhesive layer, an adhesive layer obtained by further compounding a water absorptive polymer in the non-hydrophilic adhesive can be used.

The hydrophilic adhesive which constitutes the hydrophilic adhesive layer is not particularly limited so far as it contains a hydrophilic polymer or a water-soluble polymer as the major component, has adhesion and is hydrophilic as an adhesive. Examples of the constitutional components of the hydrophilic adhesive include hydrophilic polymers (for example, polyacrylic acid), water-soluble polymers (for example, poly(sodium acrylate) and polyvinylpyrrolidone), crosslinking agents (for example, dry aluminum hydroxide and meta-silicic acid aluminic acid metal salts), softeners (for example, glycerin and propylene glycol), higher hydrocarbons (for example, soft liquid paraffin and polybutene), primary alcohol fatty acid esters (for example, isopropyl myristate), silicon-containing compounds (for example, silicone oil), fatty acid glycerin esters (for example monoglycerides), oily components (for example, vegetable oils such as olive oil), antiseptics (for example, methyl p-hydroxybenzoate and propyl p-hydroxybenzoate), solubilizing agents (for example, N-methyl-2-pyrrolidone), thickeners (for example, carboxymethyl cellulose), surfactants (for example, polyoxyethylene hardened castor oil and sorbitan fatty acid esters), hydroxycarboxylic acid (for example, tartaric acid), excipients (for example, light silicic anhydride, water absorptive polymers, and kaolin), moisturizers (for example, D-sorbitol), stabilizers (for example, sodium edetate, p-hydroxybenzoic acid esters, and tartaric acid), crosslinking type water absorptive polymers, boron compounds (for example, boric acid), and water. They may be used as an arbitrary combination.

A temporary adhering seal part is formed via a sticky layer. An adhesive which constitutes the sticky layer is a layer formed of a polymer composition which is tacky at the normal temperature and is not limited so far as it can be heat sealed after temporary adhesion.

Furthermore, the foregoing adhesives of the sticky layer can be used as the adhesive which constitutes the sticky layer as used for temporary adhesion. Of these, non-hydrophilic adhesives are preferable. With respect to the adhesive constituting the adhesive layer, it is preferable that the adhesive is well compatible with a heat seal material constituting a heat seal and that a melting point of the base polymer of the adhesive is not higher than a melting point of the heat seal material. Hot melt based adhesives are especially preferable for hot melt based bonding agents. Furthermore, in the case where the heat seal material is an olefin based raw material, preferred examples thereof include olefin based adhesives.

A bonding layer for fixing the air permeability adjusting material is constituted of a bonding agent or an adhesive which is usually used. In particular, an adhesive is useful, and the foregoing adhesives for constituting the adhesive layer can be used.

Furthermore, a method for providing a bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape.

Furthermore, in the case where an adhesive layer is employed as the hydrophilic adhesive layer, if there is a difference in a water retaining force between the hydrophilic adhesive layer and the heat generating composition molded body, transfer of water occurs via a packaging material present therebetween such as a substrate, thereby causing in-conveniences against the both. In particular, the transfer of water occurs during the storage. In order to prevent this, it is preferable that the packaging material present therebetween at least has a moisture permeability of not more than 2 $g/m^2/day$ in terms of a moisture permeability according to the Lyssy method. By using this, in the case where the heat generating body is accommodated in an outer bag as an air-impermeable accommodating bag and stored, the transfer of water can be prevented.

In the case where a hydrophilic adhesive layer is used as the adhesive layer, the moisture permeability of a moisture-proof packaging material provided between the heat generating composition molded body and the hydrophilic adhesive layer is not limited so far as the transfer of water can be prevented within the range where the exothermic performance is not affected. The moisture permeability according to the Lyssy method is usually not more than 2 $g/m^2/day$, preferably not more than 1.0 $g/m^2/day$, more preferably not more than 0.5 $g/m^2/day$, and further preferably from 0.01 to 0.5 $g/m^2/day$. These values are a value under a condition under an atmospheric pressure at 40 °C and 90 % RH. Incidentally, the moisture-proof packaging material can be used as a substrate or a covering material and may be laminated singly on a substrate, a covering material, or the like.

The moisture-proof packaging material is not limited so far as the transfer of water between the heat generating composition molded body and the hydrophilic adhesive layer can be prevented. Examples thereof include metal vapor deposited films, vapor deposited films of a metal oxide, metal foil-laminated films, EVOH (ethylene/vinyl alcohol copolymer or ethylene/vinyl acetate copolymer saponified product) based films, biaxially stretched polyvinyl alcohol films, polyvinylidene chloride coated films, polyvinylidene chloride coated films obtained by coating polyvinylidene chloride on a substrate film (for example, polypropylene), metal foils such as an aluminum foil, air-impermeable packaging materials obtained by vapor depositing or sputtering a metal (for example, aluminum) on a polyester film substrate, and packaging laminates using a transparent barrier film of a structure in which silicon oxide or aluminum oxide is provided on a flexible plastic substrate. The air-impermeable packaging materials which are used in the outer bag, etc. can also be used.

Furthermore, packaging materials such as moisture-proof packaging materials as described in JP-A-2002-200108, the disclosures of which can be incorporated herein by reference, can be used.

In the case of using a water-containing hydrophilic adhesive (for example, a gel) in the adhesive layer, in order to adjust the moisture equilibrium between the heat generating composition and the adhesive layer, the content of a reaction accelerator (for example, sodium chloride) or a substance having a water holding power (for example, a water absorptive polymer) in the heat generating composition may be adjusted within the range of from 10 to 40 % by weight, preferably from 15 to 40 % by weight, and more preferably from 15 to 30 % by weight based on the heat generating composition.

Furthermore, as the adhesive having good moisture permeability and low stimulation to the skin, water-containing adhesives (for example, hydrophilic adhesives and gels) as described in JP-A-10-265373 and JP-A-9-87173, adhesives which can be subjected to hot melt coating as described in JP-A-6-145050 and JP-A-6-199660, and rubber based adhesives as described JP-A-10-279466 and JP-A-10-182408, the disclosures of which are totally incorporated herein by reference, are useful.

The water retaining agent is not limited so far as it is able to retain water. Examples thereof include porous materials derived from plants having high capillary function and hydrophilicity such as wood meal, pulp powder, active carbon, saw dust, cotton cloth having a number of cotton fluffs, short fiber of cotton, paper dust, and vegetable materials, water-containing magnesium silicate based clay minerals such as active clay and zeolite, pearlite, vermiculite, silica based porous substances, coralline stone, and volcanic ash based substances (for example, terraballoon, *shirasu* balloon, and *taisetsu* balloon). In order to increase a water retaining ability and enhance a shape holding ability of such a water retaining agent, the water retaining agent may be subjected to a processing treatment such as baking and/or pulverization.

The water absorptive polymer is not particularly limited so far as it is a resin having a crosslinking structure and having a water absorption magnification of ion-exchanged water of 3 times or more of the dead weight. Furthermore, a water absorptive polymer the surface of which is crosslinked may be employed. Conventionally known water absorptive polymers and commercial products may also be employed.

Examples of the water absorptive polymer include poly(meth)acrylic acid crosslinked materials, poly(meth)-acrylic acid salt crosslinked materials, sulfonic group-containing poly(meth)acrylic ester crosslinked materials, polyoxyalkylene group-containing poly(meth)acrylic ester crosslinked materials, poly(meth)acrylamide crosslinked materials, crosslinked materials of a copolymer of a (meth)acrylic acid salt and a (meth)acrylamide, crosslinked materials of a copolymer of a hydroxyalkyl (meth)acrylate and a (meth)acrylic acid salt, polydioxolane crosslinked materials, crosslinked polyethylene oxide, crosslinked polyvinylpyrrolidone, sulfonated polystyrene crosslinked materials, crosslinked polyvinylpyridine, saponification products of a starch-poly (meth) acrylonitrile graft copolymer, starch-poly(meth)acrylic acid (salt) graft crosslinked copolymers, reaction products of polyvinyl alcohol and maleic anhydride (salt), crosslinked polyvinyl alcohol sulfonic acid salts, polyvinyl alcohol-acrylic acid graft copolymers, and polyisobutylene maleic acid (salt) crosslinked polymers. These water absorptive polymers may be used alone or in combination with two or more kinds thereof.

Of these water absorptive polymers, water absorptive polymers having biodegradation properties are not limited so far as they are a biodegradable water absorptive polymer. Examples thereof include polyethylene oxide crosslinked materials, polyvinyl alcohol crosslinked materials, carboxymethyl cellulose crosslinked materials, alginic acid crosslinked materials, starch crosslinked materials, polyamino acid crosslinked materials, and polylactic acid crosslinked materials.

The pH adjusting agent is not limited so far it is able to adjust the pH. Examples thereof include alkali metal weak acid salts and hydroxides and alkaline earth metal weak acid salts and hydroxides such as $Na_2CO_3$, $NaHCO_3$, $Na_3PO_4$, $Na_2HPO_4$, $Na_5P_3O_{10}$, NaOH, KOH, $Ca(OH)_2$, $Mg(OH)_2$, and $Ca_3(PO_4)_2$.

The surfactant includes anionic surfactants, cationic surfactants, nonionic surfactants, and ampholytic surfactants. Especially, nonionic surfactants are preferable, and examples thereof include polyoxyethylene alkyl ethers, alkylphenolethylene oxide adducts, and higher alcohol phosphoric acid esters.

The organosilicon compound is not limited so far as it is a compound having at least an Si-O-R bond and/or an Si-N-R bond and/or an Si-R bond. The organosilicon compound is in the form of a monomer, a lowly condensed product, a polymer, etc. Examples thereof include organosilane compounds such as methyltriethoxysilane; and dimethylsilicone oil, polyorganosiloxane, or silicone resin compositions containing the same.

The hydrophobic polymer compound is not limited so far as it is a polymer compound having a contact angle with water of 40° or more, preferably 50° or more, and more preferably 60° or more in order to improve the draining in the composition.

The shape of the hydrophobic polymer compound is not limited, and examples thereof include powdery, particulate, granular, and tablet shapes. Examples of the hydrophobic polymer compound include polyolefins such as polyethylene and polypropylene, polyesters, and polyamides.

The pyroelectric substance is not limited so far as it has pyroelectricity. Examples thereof include tourmaline, hemimorphic ores, and pyroelectric ores. Tourmaline or achroite which is a kind of tourmaline is especially preferable. Examples of the tourmaline include dravite, schorl, and elbaite.

The aggregate is not limited so far as it is useful as a filler and/or is useful for making the heat generating composition porous. Examples thereof include fossilized coral (for example, coral fossil and weathered coral fossil), bamboo charcoal, bincho charcoal, silica-alumina powders, silica-magnesia powders, kaolin, crystalline cellulose, colloidal silica, pumice, silica gel, silica powders, mica powders, clays, talc, synthetic resin powders or pellets, foamed synthetic resins such as foamed polyesters or polyurethanes, diatomaceous earth, alumina, and cellulose powder. Incidentally, it is to be noted that kaolin and crystalline cellulose are not contained in the heat generating composition of the invention.

The fibrous material is an inorganic fibrous material and/or an organic fibrous material. Examples thereof include rock wool, glass fibers, carbon fibers, metal fibers, pulps, papers, non-woven fabrics, woven fabrics, natural fibers such as cotton and hemp, regenerated fibers such as rayon, semi-synthetic fibers such as acetates, synthetic fibers, and pulverized products thereof.

The moisturizer is not limited so far as it is able to hold moisture. Examples thereof include hyaluronic acid, collagen, glycerin, and urea.

The functional substance is not limited so far as it has any function. Examples thereof include at least one kind selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, and wood vinegar.

Specific examples thereof include aromatic compounds (for example, menthol and benzaldehyde), vegetable extracts (for example, mugwort extract), crude drugs (for example, moxa), perfumes (for example, lavender and rosemary), slimming agents (for example, aminophylline and tea extract), analgesic drugs (for example, indomethacin and *dl*-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited so far as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, mold inhibitors, antimicrobial antibiotics, vitamins, antiviral agents, swelling improvers, diuretics, antihypertensives, coronary vasodilators, anti-tussive expectorants, slimming agents, anti-histamines, antiarrhythmic agents, cardiotonics, adrenocortical hormones, blood circulation promoters, local anesthetics, fat-splitting components, and mixtures thereof. However, it should not be construed that the invention is limited thereto. These drugs are used singly or in admixture of two or more kinds thereof as the need arises.

The content of such a functional substance is not particularly limited so far as it falls within the range where the effect of a medicine can be expected. However, from the viewpoints of adhesive strength as well as pharmacological effect and economy, the content of the functional substance is preferably from 0.01 to 25 parts by weight, and more preferably from 0.5 to 15 parts by weight based on 100 parts by weight of the adhesive.

Furthermore, a method for providing the adhesive layer is not limited so far as a thermal packaging body for joint surroundings can be fixed. The adhesive layer may be entirely provided or partially or intermittently provided. Examples of its shape include various shapes such as a network-like shape, a stripe-like shape, a dot-like shape, and strip-like shape.

The heat generating body of the invention is able to give various shapes, thicknesses and temperature zones and therefore, can be used for various utilities such as use for a joint, facial esthetic use, use for eyes, slimming use, use for heating or warming a dripping solution, use for a wet compress pack, use for a medical body warmer, use for a neck, use for a waist, use for a mask, use for a glove, use for hemorrhage, use for relaxation of symptoms such as shoulder pain, muscular pain, and menstrual pain, use for a cushion, use for heating or warming a human body during the operation, use for a thermal sheet, use for thermally volatilizing an aroma, use for an abdomen, insecticidal use by thermal volatilization, and use for treating cancer in addition to common warming of a human body. In addition, the heat generating body of the invention can be used for heating or warming machines, pets, etc.

For example, in the case of using for relaxation of symptoms, the heat generating body of the invention is applied directly in a necessary site of the body or indirectly via a cloth, etc. Furthermore, in the case of using for heating or warming a human body during the operation, a method for using the heat generating body of the invention includes the following methods.

(1) The heat generating body is directly applied to a body requiring heating or warming.
(2) The heat generating body is fixed on a covering, etc. and covered on the body.
(3) The heat generating body is fixed on a cushion to be placed beneath the body, etc.
(4) The heat generating body is used as a covering or a cushion which is a product having the heat generating body provided therein in advance.
Incidentally, examples of the pain of muscles or bones include acute muscle pain, acute bone pain, acute reference pain, previous muscle pain, previous bone pain, chronic reference pain, and join pain of knee, elbow, etc.
The holding time is not limited but is preferably from 20 seconds to 24 hours, more preferably from one hour to 24 hours, and further preferably from 8 hours to 24 hours.
The holding temperature is preferably from 30 to 50 °C, more preferably from 32 to 50 °C, further preferably from 32 to 43 °C, still further preferably from 32 to 41 °C, and even further preferably from 32 to 39 °C.

The invention will be specifically described below with reference to the Examples. Next, the invention will be specifically described with reference to the Examples, but it should not be construed that the invention is limited thereto.

[Brief Description of the Drawings]

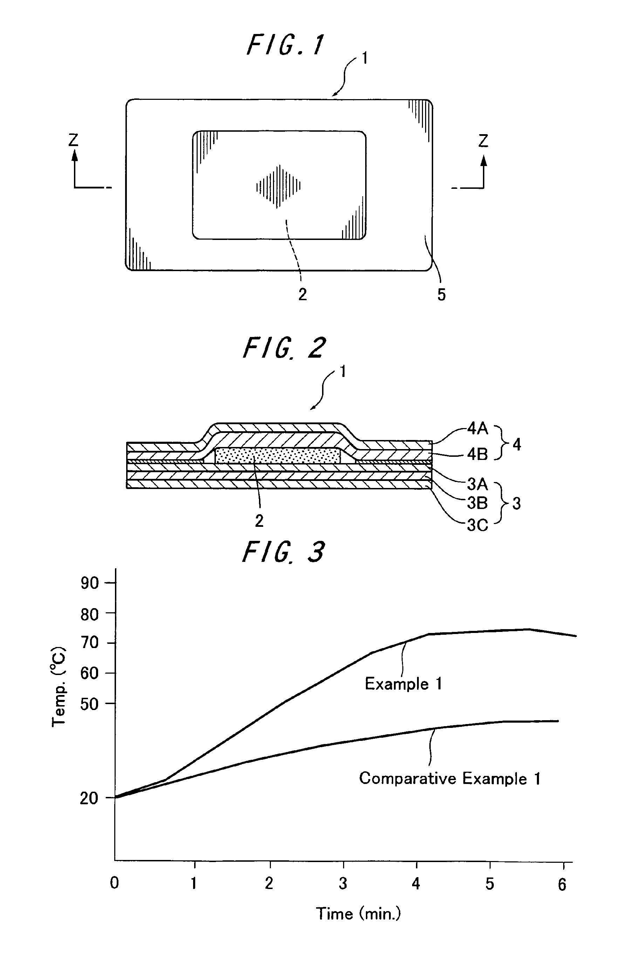

[Fig. 1] is a plan view of an embodiment of the heat generating body of the invention.
[Fig. 2] is a cross-sectional view along the line Z-Z of the same.
[Fig. 3] is a diagram of exothermic characteristics of the heat generating composition of Example 1 and Comparative Example 1.

[Fig. 4] is a diagram of exothermic characteristics of the heat generating body of Examples 2 and Comparative Example 2.

[Fig. 5] is a plan view of another embodiment of the heat generating body of the invention.

[Fig. 6] is a plan view of a filter paper for the measurement of water mobility value in the invention.

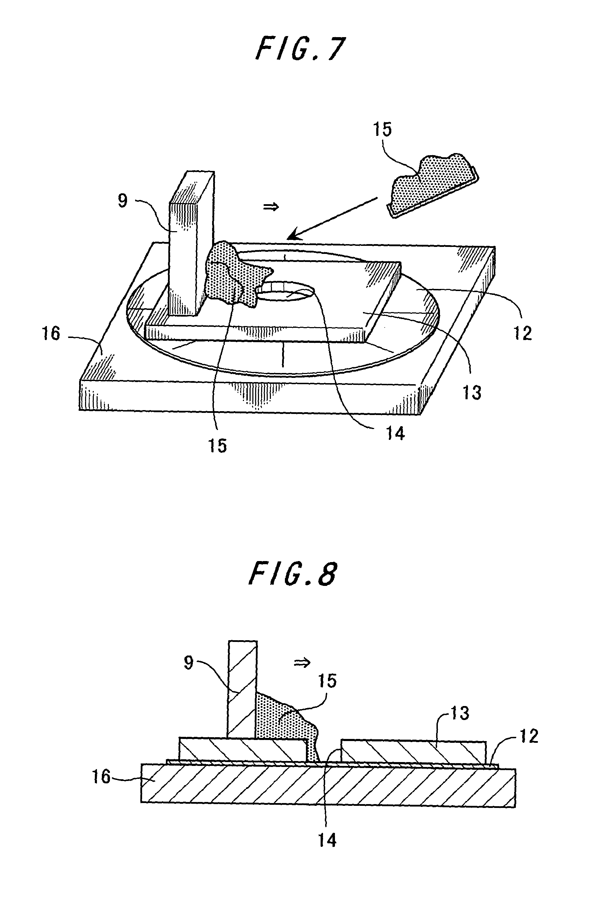

[Fig. 7] is an oblique view for explaining the measurement of water mobility value in the invention.

[Fig. 8] is a cross-sectional view for explaining the measurement of water mobility value in the invention.

[Fig. 9] is a cross-sectional view for explaining the measurement of water mobility value in the invention.

[Fig. 10] is a plan view of a filter paper after carrying out the measurement of water mobility value in the invention.

[Description of Reference Numerals and Signs]

1: Heat generating body
1C: Sectioned part (seal part)
2: Heat generating composition molded body
2': Heat generating composition molded body (sectional exothermic part)
3: Substrate
4: Covering material
8: Perforation
9: Pushing plate
10: Flat plate
11: Non-water absorptive film (polyethylene film, etc.)
12: Filter paper in which eight lines are drawn radiating from the central point with an interval of 45°
13: Die plate
14: Hole
15: Sample
16: Stainless steel plate
17: Distance to the oozed-out locus of water or aqueous solution
18: Position corresponding to a hollow cylindrical hole on filter paper

[Examples]

(Example 1)

A stirring type batchwise oxidizing gas contact treatment device consisting of a mixer equipped with a rotary blade for stirring was used as an oxidizing gas contact treatment device, and air was used as an oxidizing gas. First of all, a reaction mixture consisting of 100 parts by weight of a reduced iron powder (particle size: not more than 300 $\mu$m), 3.0 parts by weight of active carbon (particle size: not more than 300 $\mu$m), and 10 parts by weight of 11 % salt water and having a water mobility value of less than 0.01 was charged in the stirring type batchwise oxidizing gas contact treatment device. Next, in the state that the upper portion of the oxidizing gas contact treatment device was opened to air, the reaction mixture was subjected to self heat generation with stirring under circumstances at 20 °C and contact treated with an oxidizing gas at a maximum exothermic temperature of 68 °C until the exothermic temperature reached 35 °C, thereby obtaining a contact treated reaction mixture. With respect to the iron powder of the contact treated reaction mixture, an integrated intensity ratio was determined from an integrated intensity of peaks (at 58.28, 64.92 and 82.22 ($2\theta$/deg)) of a (110) plane of iron ($\alpha$Fe) and an integrated intensity of peaks (at 35.24, 41.59, 60.95, 72.70 and 76.51 ($2\theta$/deg)) of a (220) plane of FeO (wustite) by using an X-ray diffraction device, from which was then determined an amount of wustite.

The amount of wustite of the reaction mixture was 10 %.

Then, 11 % salt water was mixed in the foregoing contact treated reaction mixture to obtain a heat generating composition having a water mobility value of 10.

(Comparative Example 1)

A heat generating composition having a water mobility value of 10 was prepared in the same manner as in Example 1, except that the contact treatment with an oxidizing gas was not carried out.

Each of the heat generating compositions as obtained in Example 1 and Comparative Example 1 was subjected to an exothermic test, thereby obtaining the results as shown in Fig. 3. Comparative Example 1 was deteriorated in exothermic rising properties.

(Example 2)

A stirring type batchwise oxidizing gas contact treatment device consisting of a mixer equipped with a rotary blade for stirring was used as an oxidizing gas contact treatment device, and air was used as an oxidizing gas. First of all, a reaction mixture consisting of 100 parts by weight of a reduced iron powder (particle size: not more than 300 $\mu$m), 5.2 parts by weight of active carbon (particle size: not more than 300 $\mu$m), 2.3 parts by weight of a wood meal (particle size: not more than 300 $\mu$m), 2. 3 parts by weight of a water absorptive polymer (particle size: not more than 300 $\mu$m), 0.2 parts by weight of calcium hydroxide, 0.7 parts by weight of sodium sulfite, and 10 parts by weight of 11 % salt water and having a water mobility value of less than 0.01 was charged in the stirring type batchwise oxidizing gas contact treatment device. Next, in the state that the upper portion of the oxidizing gas contact treatment device was opened to air, the reaction mixture was subjected to self heat generation with stirring under circumstances at 20 °C and contact treated with an oxidizing gas at a maximum exothermic temperature of 68 °C until the exothermic temperature reached 35 °C, thereby obtaining a contact treated reaction mixture. With respect to the iron powder of the contact treated reaction mixture, an integrated intensity ratio was determined from an integrated intensity of peaks of a (110) plane of iron ($\alpha$Fe) and an integrated intensity of peaks of a (220) plane of FeO (wustite) by using an X-ray diffraction device, from which was then determined an amount of wustite. The amount of wustite of the reaction mixture was 10 %. Next, 11 % salt water was mixed in the foregoing contact treated reaction mixture to obtain a heat generating composition having a water mobility value of 8.

This heat generating composition was subjected to an exothermic test of heat generating composition. As a result, the temperature reached about 50 °C (an average value of five samples) after 3 minutes.

Furthermore, the heat generating composition was tested for moldability. As a result, even after separating a trimming die from a heat generating composition molded body, the heat generating composition molded body was free from a loss of shape, and collapsed pieces of the heat generating composition molded body were not generated in the surroundings of the heat generating composition molded body.

Next, as illustrated in Figs. 1 and 2, by using the heat generating composition of Example 2, a heat generating composition molded body 2 was laminated on a polyethylene film 3A of an air-impermeable substrate 3 in which an adhesive layer 3B provided with a separator 3C was provided on the polyethylene film 3A by a trimming die having a rectangular cavity of 2 mm in thickness, 110 mm in length and 80 mm in width. In addition, an air-permeable covering material 4 made of a laminate of a nylon-made non-woven fabric 4A and a porous film 4B was superimposed thereon such that the surface of the polyethylene film 3A and the surface of the porous film 4B were brought into contact with each other. The surroundings were heat sealed in a seal width of 8 mm and then cut to produce a rectangular flat heat generating body 1 of 130 mm in length, 100 mm in width and 8 mm in seal width.

Even after separating the trimming die from the heat generating composition molded body 2, the laminate was free from a loss of shape, and collapsed pieces of the heat generating composition molded body 2 were not generated in the surroundings of the heat generating composition molded body 2. Also, sealing could be completely carried out without causing incorporation of collapsed pieces of the heat generating composition molded body 2 into the seal part, and seal failure did not occur. Incidentally, the air permeability of the covering material 4 was 370 g/m$^2$/24 hr in terms of a moisture permeability by the Lyssy method.

Next, the heat generating body was sealed and accommodated in an air-impermeable outer bag and then allowed to stand at room temperature for 24 hours.

After 24 hours, the heat generating body was taken out from the outer bag and subjected to an exothermic test by the body. As a result, it was felt warm after 3 minutes, and thereafter, the warmth was continued for 10 hours or more.

(Comparative Example 6)

A heat generating composition was obtained in the same manner as in Example 2, except that the contact treatment with an oxidizing gas was not carried out, from which was then obtain a heat generating body. The heat generating body was subjected to an exothermic test by the body. As a result, it took 6 minutes until it was felt warm.

With respect to Example 2 and Comparative Example 2, the exothermic test of heat generating body was carried out. As a result, as shown in Fig. 4, in the case of Example 2, the temperature was 34 °C after 3 minutes and 37 °C after 10 minutes, respectively. However, in the case of Comparative Example 2, the temperature was 22 °C after 30 minutes and 34 °C after 10 minutes, respectively. The heat generating body using the heat generating composition of the invention was excellent with respect to the exothermic rising properties.

(Example 3)

By using the heat generating composition of Example 2, a heat generating composition molded body 2' (sectional exothermic part) of a rectangular parallelepiped of 2 mm in thickness, 115 mm in length and 80 mm in width was laminated in the side of a polyethylene film of an air-impermeable substrate in which an adhesive layer provided with a separator was provided on a polyethylene film by force-through molding using a trimming die having a thickness of 2 mm and comprising 9 square cavities having a length of one side of 15 mm as illustrated in Fig. 5. In addition, an air-permeable covering material made of a laminate of a nylon-made non-woven fabric and a polyethylene-made porous film was superimposed thereon such that the surface of the foregoing polyethylene film and the surface of the porous film were brought into contact with each other. The periphery of the heat generating composition molded body 2' (sectional exothermic part) was heat sealed in a seal width of 8 mm to provide a seal part 1C, from which was then produced a rectangular irregular heat generating body 1 of 135 mm in length, 100 mm in width and 8 mm in seal width. Incidentally, in Fig. 5, the numeral 8 represents a perforation from which cutting by hand is possible. Furthermore, the air permeability of the covering material was 370 g/m$^2$/24 hr in terms of a moisture permeability by the Lyssy method. The heat generating body was sealed and accommodated in an air-impermeable outer bag and then allowed to stand at room temperature for 24 hours. As a result of an exothermic test by the body, it was felt warm after 3 minutes, and thereafter, the warmth was continued for 10 hours or more.

**Claims**

1. An active iron powder to be contained in a heat generating composition capable of generating heat upon contact with air, **characterized in that** an amount of wustite to be contained in an iron powder is from 5.01 to 50 % by weight in terms of an X-ray peak intensity ratio to iron.

2. The active iron powder according to claim 1, **characterized in that** the active iron powder is at least one member selected from a reduced iron powder, an atomized iron powder, and an iron powder comprising particles, a surface of each of which is at least partially covered by a conductive carbonaceous substance.

3. A heat generating composition containing as essential components containing an iron active powder according to claim 1, a carbon component, a reaction accelerator and water, **characterized in that** the active iron powder accounts for from 30 to 100 % by weight of the iron powder in the heat generating composition.

4. A heat generating body, **characterized by** containing the heat generating composition according to claim 3.

5. The heat generating body according to claim 4, **characterized in that** the heat generating body has fixing means in at least a part thereof.

6. The heat generating body according to claim 5, **characterized in that** the fixing means is an adhesive layer; and that the adhesive layer contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

## FIG. 1

## FIG. 2

## FIG. 3

## FIG. 4

## FIG.5

## FIG.6

*FIG.7*

*FIG.8*

## FIG.9

## FIG.10

## INTERNATIONAL SEARCH REPORT

| International application No. |
| --- |
| PCT/JP2005/013000 |

**A. CLASSIFICATION OF SUBJECT MATTER**
Int.Cl$^7$ A61F7/08, C09K5/16

According to International Patent Classification (IPC) or to both national classification and IPC

**B. FIELDS SEARCHED**

Minimum documentation searched (classification system followed by classification symbols)
Int.Cl$^7$ A61F7/08, C09K5/16

Documentation searched other than minimum documentation to the extent that such documents are included in the fields searched
Jitsuyo Shinan Koho          1922-1996   Jitsuyo Shinan Toroku Koho    1996-2005
Kokai Jitsuyo Shinan Koho    1971-2005   Toroku Jitsuyo Shinan Koho    1994-2005

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 10-17907 A  (Dowa Iron Powder Co., Ltd.), 20 January, 1998 (20.01.98), Full text; all drawings (Family: none) | 1-6 |
| A | JP 2002-155273 A  (Kaoru USUI), 28 May, 2002 (28.05.02), Full text; all drawings (Family: none) | 1-6 |
| A | JP 7-265347 A  (Yukio KOMATSU), 17 October, 1995 (17.10.95), Full text; all drawings (Family: none) | 1-6 |

☒  Further documents are listed in the continuation of Box C.          ☐  See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 25 August, 2005 (25.08.05) | 13 September, 2005 (13.09.05) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

<div align="center">**INTERNATIONAL SEARCH REPORT**</div>

| International application No. |
| --- |
| PCT/JP2005/013000 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2003-102761 A  (Kao Corp.),<br>08 April, 2003 (08.04.03),<br>Full text; all drawings<br>& US 2005/0000827 A1      & EP 1437111 A1<br>& WO 2003/028597 A1      & CN 1561187 A | 1-6 |
| A | JP 2004-149476 A  (Maikoru Purodakutsu Kabushiki Kaisha),<br>27 May, 2004 (27.05.04),<br>Par. Nos. [0009], [0025]; Fig. 1<br>& US 2004/0197502 A1      & EP 1557146 A1<br>& WO 2004/039294 A1      & CN 1571655 A | 5-6 |

Form PCT/ISA/210 (continuation of second sheet) (January 2004)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 53060885 A
- JP 57010673 A
- JP 11508786 T
- JP 2002200108 A
- JP 10265373 A
- JP 9087173 A
- JP 6145050 A
- JP 6199660 A
- JP 10279466 A
- JP 10182408 A